# EUROPEAN PATENT APPLICATION

(11) **EP 4 328 325 A1**
(43) Date of publication of application: **28.02.2024**
(21) Application number: 21937375.0
(22) Date of filing: 23.04.2021
(51) Int. Cl.: C12Q 1/6886, C12Q 1/6858, C12N 15/11

(54) **COMPOSITION, KIT, AND APPLICATION FOR DETECTION OF COLORECTAL CANCER**

(71) Applicant: BGI Genomics Co., Limited, Guangdong 518083 (CN)
(72) Inventor: WANG, Yuying, Shenzhen, Guangdong 518083 (CN); PENG, Jiaxi, Shenzhen, Guangdong 518083 (CN); SUN, Jianlong, Shenzhen, Guangdong 518083 (CN); LI, Zhilong, Shenzhen, Guangdong 518083 (CN); JIANG, Ruijingfang, Shenzhen, Guangdong 518083 (CN); ZHENG, Jianchao, Shenzhen, Guangdong 518083 (CN); ZHU, Shida, Shenzhen, Guangdong 518083 (CN)
(74) Representative: Winter, Brandl - Partnerschaft mbB
(86) International application number: PCT/CN2021/089368
(87) International publication number: WO 2022/222146

(57) **Abstract**

Provided are a composition, kit, and application for the detection of colorectal cancer. The provided composition contains reagents for detecting methylation status of *ADHFE1* gene, *PPP2RSC* gene, and *SDC2* gene. The composition can be used for detecting colorectal cancer with high sensitivity and specificity.

## Description

### FIELD

The present application relates to the field of biotechnology, and particularly, to a composition, kit, and application for the detection of colorectal cancer.

### BACKGROUND

Colorectal cancer, which primarily affects the colon and rectum in the lower gastrointestinal tract, is a common malignant tumor. According to the latest statistics, it ranks third in males and second in females among new cancer cases worldwide, and it is the fourth leading cause of cancer-related deaths in males and third in females (Bray *et al.* 2018). In China, colorectal cancer ranks third in terms of new cancer cases (380,000 people) and fifth in terms of dead cases (187,000 people) (Zheng Rongshou *et al.,* 2019). Therefore, there is an urgent need for colorectal cancer prevention and intervention. Colorectal cancer develops slowly and generally goes through the process of polyp, adenoma, and finally malignancy. It can take as long as 5 to 10 years to develop from adenoma to cancer. Early intervention during the stages of colorectal cancer can significantly reduce mortality. The five-year survival rate for patients with stage I colorectal cancer can exceed 90%, while the five-year survival rate for patients with stage IV is less than 20% (Marzieh Araghi *et al.,* 2020).

Traditional early screening techniques for colorectal cancer include colonoscopy, fecal occult blood test, and tumor markers CEA and CA19-9. However, these techniques all have certain limitations. Colonoscopy is considered the gold standard for colorectal cancer diagnosis. Through the insertion of a scope and a light source from the anus, the doctor can visualize the rectum, sigmoid colon, and other parts of the colon in real-time on a monitor. Colonoscopy provides clear and direct imaging, enabling the observation of various lesions such as cancer, polyps, ulcers, and bleeding. Additionally, it allows for the removal of polyps during the procedure. However, colonoscopy is an invasive procedure that requires complex preparation, including dietary restrictions and bowel preparation. Compliance among individuals undergoing colonoscopy is often low. In addition, it requires specialized equipment and personnel, with trained professionals and anesthesiologists in a hospital setting. Some subjects may experience discomfort and risk of complications (3-5 cases/1000 persons). The fecal occult blood test is a non-invasive method for detecting the presence of blood components (hemoglobin) in the stool, indicating possible gastrointestinal bleeding and assessing the risk of colorectal cancer. Such a method is fast and simple, and the compliance of the examinee is high. However, this detection may be affected to show false positives by the diet of liver or blood products, and green leafy vegetables. Additionally, the sensitivity is low for early colorectal cancer with minimal bleeding which may result in missed diagnoses. Tumor markers like CEA are broad-spectrum markers with low specificity, which may produce more false positives, and their sensitivity is also inferior. Therefore, it is necessary to establish an accurate, simple, and cost-effective method for colorectal cancer early screening.

DNA methylation is an important mechanism of gene expression regulation, capable of modulating gene expression and silencing. It has a significant impact on the occurrence and development of tumors. Aberrant methylation of cancer-associated genes often occurs in the early stage of cancer development, making DNA methylation a potential biomarker for early screening. Human fecal samples include DNA information due to the colon epithelium constantly shedding cells into the intestinal lumen with cell turnover, which carries information such as methylation. By detecting and analyzing this information, it is possible to infer the likelihood of an individual having colorectal cancer. Certain genes have been identified as methylation markers for the detection of colorectal cancer in feces, e.g., SFRP2, SEPT9, NDRG4, and SDC2, etc. (Hannes M Müller *et al.* 2004; Jie Chen *et al.* 2019), but their performance has not yet fully met clinical requirements. For example, the sensitivity and specificity of SFRP2 for colorectal cancer is only 77% (Hannes M Müller *et al.* 2004).

### SUMMARY

The present disclosure aims to solve at least one of the technical problems in the related art to some extent and provides a composition, kit, and application for the detection of colorectal cancer. The provided composition can be used to detect colorectal cancer with high specificity and sensitivity, such that it can assist in clinical detection and enrich the diagnosis and detection of colorectal cancer.

Specifically, the present disclosure provides the following technical solutions.

In a first aspect, the present disclosure provides a composition for colorectal cancer detection, which includes reagents for detecting the methylation status of *ADHFE1* gene, *PPP2R5C* gene, and *SDC2* gene.

In a second aspect, the present disclosure provides a kit for colorectal cancer diagnosis. The kit includes a composition for colorectal cancer detection according to the first aspect described above.

In a third aspect, the present disclosure provides a method for determining the methylation status of genes in a provided sample. The genes include an *ADHFE1* gene, a *PPP2R5C* gene, and an *SDC2* gene. The method includes: (1) extracting a genomic DNA from the sample; (2) obtaining a conversion product by performing methylation conversion on the genomic DNA, wherein a methylated site exhibits a difference from and a non-methylated site after the treatment; and (3) utilizing a primer set and probes to perform fluorescent quantitative PCR detection on the conversion product to determine the gene methylation status in the sample.

In a fourth aspect, the present disclosure provides isolated nucleic acid sequences including a primer set and probes. The primer set includes: (a) at least one of sequences as set forth in SEQ ID NO: 3 to SEQ ID NO: 40; (b) at least one of sequences as set forth in SEQ ID NO: 41 to SEQ ID NO: 60; and (c) at least one of sequences as set forth in SEQ ID NO: 61 to SEQ ID NO: 98. The probes include: at least one of sequences as set forth in SEQ ID NO: 100 to SEQ ID NO: 112; at least one of sequences as set forth in SEQ ID NO: 113 to SEQ ID NO: 127; and at least one of sequences as set forth in SEQ ID NO: 128 to SEQ ID NO: 142.

In a fifth aspect of the present disclosure, the present disclosure provides a method for assessing a disease state of colorectal cancer in a subject. The method includes: a) providing a sample of the subject, the sample comprising target nucleic acids of the subject, and the target nucleic acids comprising nucleic acids from *ADHFE1* gene, *PPP2RSC* gene, and *SDC2* gene; b) assessing a methylation status of the target nucleic acids; and c) assessing the diseased state of colorectal cancer in the subject based on the methylation status of the target nucleic acids.

Additional aspects and advantages of the present disclosure will be set forth in part in the description which follows and, in part, will be obvious from the description, or may be learned by practice of the present disclosure.

### BRIEF DESCRIPTION OF DRAWINGS

The foregoing and/or additional aspects and advantages of the present disclosure will become apparent and readily appreciated from the following description of the embodiments, taken in conjunction with the accompanying drawings.
FIG. 1 is a schematic diagram of methylation levels in colorectal tumor tissue and adjacent normal tissue samples in the TCGA database according to an embodiment of the present disclosure.
FIG. 2 is a graph of fluorescent quantitative PCR amplification curve of a positive standard of Example 2 according to the present disclosure.
FIG. 3 is a graph of fluorescent quantitative PCR amplification curve of a negative standard of Example 2 according to the present disclosure.
FIG. 4 is a detection flow chart of a kit for diagnosing colorectal cancer provided in Example 3 according to the present disclosure.
FIG. 5 is a ROC graph of colorectal cancer samples detected for each gene in Example 3 according to the present disclosure.

### DETAILED DESCRIPTION

Reference will now be made in detail to the embodiments of the present disclosure, it should be understood that the embodiments described are illustrative and are intended to explain, rather than limiting, the present disclosure. In the process of describing the solutions provided by the present disclosure, the relevant terms in the text are defined and explained. These definitions and explanations are only for the convenience of understanding the solutions, and they should not be regarded as limitations on the protection solutions of the present disclosure.

In an aspect of the present disclosure, the present disclosure provides a composition for detecting colorectal cancer. The composition includes reagents for detecting methylation status of *ADHFE1* gene, *PPP2RSC* gene, and *SDC2* gene. The composition described herein does not require that two or more substances are in contact and mixed with each other, but it means that the composition is applied in the same environment at the time of use. For example, during detecting colorectal cancer, the methylation status of the *ADHFE1* gene, the *PPP2RSC* gene, and the *SDC2* gene are simultaneously detected.

The methylation modifications of the *ADHFE1* gene, *PPP2RSC* gene, and *SDC2* gene, as mentioned herein, are closely related to the occurrence and development of cancer, and they exhibit significant differences between normal tissue and tumor tissue. For example, the *SDC2* gene, the *ADHFE1* gene, and the *PPP2R5C* gene are present with high levels of methylation in carcinoma tissues. The *ADHFE1* gene encodes a transhydrogenase that is involved in intracellular metabolism and other physiological activities, and hypermethylation of *ADHFE1* had been shown to promote the proliferation of colorectal cancer cell via modulating cell cycle progression (Hu YH *et al.* 2019). *ADHFE1* is hypermethylated in advanced adenoma and carcinoma tissues (Tae CH *et al.* 2013), and the significant differences in *ADHFE1* methylation level among normal, adenoma, and carcinoma tissues make it an ideal marker in colorectal cancer detection (Naumov VA *et al.* 2013. Fan, J *et al.* 2020). The *PPP2RSC* gene encodes a regulatory subunit of PP2A phosphatase and is a negative regulator of cell growth and proliferation (Veerle JANSSENS *et al.* 2001). It reported that its encoded protein can regulate the dephosphorylation of p53 protein in response to DNA injury, thereby inhibiting the growth of intestinal cancer cells (Li HH *et al.* 2007). The hypermethylation status of the *PPP2RSC* gene is closely related to the occurrence and development of intestinal cancer, and it can be used as a potential marker for the detection of intestinal cancer (Galamb O *et al.* 2016). The *SDC2* gene encodes an intrinsic membrane protein that is involved in cell division, cell migration, and interstitial interactions (Kim JH *et al.* 2018). Several studies have identified that the DNA methylation level of *SDC2,* which increased as colorectal cancer progressed, was significantly higher in colorectal cancer tissues than adjacent normal tissues and was an effective marker for the diagnosis of colorectal cancer (Oh TJ *et al.* 2017).

At present, the *SDC2* gene has become a more reliable standard for the diagnosis and detection of colorectal cancer. In the present disclosure, by combining the *SDC2* gene with the *ADHFE1* gene and *PPP2R5C* gene, the sensitivity and specificity are higher than those of *SDC2* gene detection alone. Furthermore, the combination of the *SDC2* gene, *ADHFE1* gene, and *PPP2R5C* gene has higher specificity and sensitivity for colorectal cancer diagnosis compared with other genes such as *NDRG4, BMP3,* etc. For example, in the present disclosure, the sensitivity may be more than 85%, and the specificity may be more than 90%. In an example, the sensitivity may be more than 87%, even more than 90%, and the specificity may be more than 90%, even more than 94%. Moreover, it is more likely to show differentiation between colorectal cancer samples and normal samples.

In at least some embodiments, the reagents include a primer set and probes. As provided herein, the term "primer" refers to a short nucleic acid sequence having a free hydroxyl group at 3'-end and capable of forming base pairs with a complementary template, the primer serving as an origin of template strand replication. Under appropriate buffer and temperature conditions, the primer can initiate DNA synthesis in the presence of various nucleoside triphosphates and polymerizing reagents such as DNA polymerase or reverse transcriptase.

The term "probe" refers to a fragment of a polynucleotide, such as RNA or DNA, which is capable of specifically binding to the mRNA or complementary DNA (cDNA) of a particular gene and has a length of several to several hundred base pairs. Since the probe is labeled, the probe can be used to detect the presence or level of expression of the target mRNA or cDNA to be bound.

In some embodiments, the primer set includes: (a-1) at least one of the sequences as set forth in SEQ ID NO: 3 to SEQ ID NO: 40 (e.g., at least one pair of these sequences); (b-1) at least one of the sequences as set forth in SEQ ID NO: 41 to SEQ ID NO: 60 (e.g., at least one pair of these sequences); and (c-1) at least one of the sequences as set forth in SEQ ID NO: 61 to SEQ ID NO: 98 ((e.g., at least one pair of these sequences).

In some embodiments, probes include: (a-2) at least one of the sequences as set forth in SEQ ID NO: 100 to SEQ ID NO: 112; (b-2) at least one of the sequences as set forth in SEQ ID NO: 113 to SEQ ID NO: 127; and (c-2) at least one of the sequences as set forth in SEQ ID NO: 128 to SEQ ID NO: 142.

In at least some embodiments, the primer set includes (a-1) at least one pair of the sequences as set forth in SEQ ID NO: 3 to SEQ ID NO: 40; (b-1) at least one pair of the sequences as set forth in SEQ ID NO: 41 to SEQ ID NO: 60; and (c-1) at least one pair of the sequences as set forth in SEQ ID NO: 61 to SEQ ID NO: 98. In at least some embodiments, probes include: (a-2) at least one of the sequences as set forth in SEQ ID NO: 100 to SEQ ID NO: 112; (b-2) at least one of the sequences as set forth in SEQ ID NO: 113 to SEQ ID NO: 127; and (c-2) at least one of the sequences as set forth in SEQ ID NO: 128 to SEQ IDNO: 142.

At least one pair of the above-mentioned sequences refers to a pair of primers, i.e., any forward primer and any reverse primer of a certain identical gene (i.e., *ADHFE1* gene, *PPP2R5C* gene, or *SDC2* gene mentioned herein). It should be noted that, when the primers and probes for the same gene are used in combination, any forward primer of a certain gene and a reverse primer located downstream of the forward primer as well as the probes located between the forward and reverse primers can be used in combination, for implementing the amplification and detection of colorectal cancer-related genes. Those skilled in the art can use combinations of primers and probes to align sequences to the genome based on the sequences provided herein. For example, the forward primer numbered SEQ ID NO: 7 or SEQ ID NO: 11 and the reverse primer numbered SEQ ID NO: 8 or SEQ ID NO: 24 can be used in combination as a pair of primers, and they can be used in combination with the probes numbered SEQ ID NO: 115, SEQ ID NO: 114, SEQ ID NO: 125, or SEQ ID NO: 126, as desired.

In some embodiments, the primer set further includes sequences as set forth in SEQ ID NO: 1 to SEQ ID NO: 2, and the probes further include a sequence as set forth in SEQ ID NO: 99. The sequences as set forth in SEQ ID NO: 1 to SEQ ID NO: 2 and SEQ ID NO: 99 can be used for PCR amplification and detection of the internal reference gene *GAPDH* gene.

The respective sequences are listed in Table 1. The primer sets and probes are designed with respect to the methylation-modified sequences of *ADHFE1, PPP2RSC,* and *SDC2* genes, and they can specifically recognize the methylation-modified sequences. The *GAPDH* gene is used as an internal reference, the amplification of the internal reference gene is not affected by methylation modification, enabling the template number in the sample to be quantified. By calculating the difference between Ct value of the target gene and Ct value of the internal reference gene, the methylation degree of the sample can be determined, and it is helpful for detecting colorectal cancer. 5'-end of the mentioned probe may be bound to a FAM, VIC, ROX, and CY5 fluorophore, while 3'-end may be bound to a quencher such as MGB. The fluorophore and quencher are those commonly used in the related art.

**[Table 1] List of sequences of primer sets and probes**

| Sequence Number | Sequence | Function |
|---|---|---|
| SEQ ID NO: 1 | TTTAGGAGTGAGTGGAAGATAGA | *GAPDH* forward primer 1 |
| SEQ ID NO: 2 | AAACCACACCATCCTAATTACCT | *GAPDH* reverse primer 1 |
| SEQ ID NO: 3 | TCGTTGGGGTAGTTGGC | *ADHFE1* forward primer 1 |
| SEQ ID NO: 4 | CCTATCTAAACCTCAAACCAATCG | *ADHFE1* reverse primer 1 |
| SEQ ID NO: 5 | GTTTTGGGTTTTTATCGCGC | *ADHFE1* forward primer 2 |
| SEQ ID NO: 6 | GCCCTAAAAAACTACATCGCG | *ADHFE1* reverse primer 2 |
| SEQ ID NO: 7 | GATGGTGCGAGCGTC | *ADHFE1* forward primer 3 |
| SEQ ID NO: 8 | CTATCTAAACCTCAAACCAATCG | *ADHFE1* reverse primer 3 |
| SEQ ID NO: 9 | GGTAATTTTAAGGGTGGATG | *ADHFE1* forward primer 4 |
| SEQ ID NO: 10 | CTCAAAACCATTTTCCCAC | *ADHFE1* reverse primer 4 |
| SEQ ID NO: 11 | AAGTAAGGAGATTTAAGGTAG | *ADHFE1* forward primer 5 |
| SEQ ID NO: 12 | CTCAAAACCATTTTCCCAC | *ADHFE1* reverse primer 5 |
| SEQ ID NO: 13 | GGGTTATTTGTTTAAGTTTTAAGT | *ADHFE1* forward primer 6 |
| SEQ ID NO: 14 | CCCCAAATCCTCAACA | *ADHFE1* reverse primer 6 |
| SEQ ID NO: 15 | GTTGTAGTTGTTTTAGTAAGT | *ADHFE1* forward primer 7 |
| SEQ ID NO: 16 | TTAAAACTTAAACAAATAACCC | *ADHFE1* reverse primer 7 |
| SEQ ID NO: 17 | GATTGGTTTGAGGTTTAGA | *ADHFE1* forward primer 8 |
| SEQ ID NO: 18 | AATCCTCTTCCCTCCT | *ADHFE1* reverse primer 8 |
| SEQ ID NO: 19 | GATTGGTTTGAGGTTTAGA | *ADHFE1* forward primer 9 |
| SEQ ID NO: 20 | TTACAATTACCTCAACAAATAC | *ADHFE1* reverse primer 9 |
| SEQ ID NO: 21 | GGTAATTTTAAGGGTGGATGGTG | *ADHFE1* forward primer 10 |
| SEQ ID NO: 22 | TCACCTATCTAAACCTCAAACCA | *ADHFE1* reverse primer 10 |
| SEQ ID NO: 23 | AGGTTTAGATAGGTGATTTCGC | *ADHFE1* forward primer 11 |
| SEQ ID NO: 24 | AAACTACCCGCCTCCC | *ADHFE1* reverse primer 11 |
| SEQ ID NO: 25 | TTGGCGTTTTGGTTTTTATTTC | *ADHFE1* forward primer 12 |
| SEQ ID NO: 26 | CCTATCTAAACCTCAAACCAATCG | *ADHFE1* reverse primer 12 |
| SEQ ID NO: 27 | GATTTAAGGTAGAATTCGAGGTTTAC | *ADHFE1* forward primer 13 |
| SEQ ID NO: 28 | CACGAAATAAAAACCAAAACG | *ADHFE1* reverse primer 13 |
| SEQ ID NO: 29 | CGTAGTTTTTTGGGTGTGATTC | *ADHFE1* forward primer 14 |
| SEQ ID NO: 30 | AAAACGATATCCAAATTCTCCG | *ADHFE1* reverse primer 14 |
| SEQ ID NO: 31 | CGTATTTTGTGATTTCGTTGTTTC | *ADHFE1* forward primer 15 |
| SEQ ID NO: 32 | AAAAATTCGAAAAATTTAAATACCG | *ADHFE1* reverse primer 15 |
| SEQ ID NO: 33 | CGTTTGTTTATTCGTTTCGC | *ADHFE1* forward primer 16 |
| SEQ ID NO: 34 | CGCCGCTAAAACAACTAACG | *ADHFE1* reverse primer 16 |
| SEQ ID NO: 35 | CGTTTTGAGAGGTTGTATCGC | *ADHFE1* forward primer 17 |
| SEQ ID NO: 36 | AAAAATTCGAAAAATTTAAATACCG | *ADHFE1* reverse primer 17 |
| SEQ ID NO: 37 | GTTTAGGGCGGTATTTAAATTTTTC | *ADHFE1* forward primer 18 |
| SEQ ID NO: 38 | ATATAACCCGCTCAAATCCTACG | *ADHFE1* reverse primer 18 |
| SEQ ID NO: 39 | TGTATCGCGTATTTTGTGATTTC | *ADHFE1* forward primer 19 |
| SEQ ID NO: 40 | CCGCTTACTTTCAAAAATTCG | *ADHFE1* reverse primer 19 |
| SEQ ID NO: 41 | GGTTTTCGTTGTTTCGTTTC | *PPP2RSC* forward primer 1 |
| SEQ ID NO: 42 | CCAAATCGACGTCCTCCG | *PPP2RSC* reverse primer 1 |
| SEQ ID NO: 43 | GGTTTTAGGTCGGGGTTTC | *PPP2RSC* forward primer 2 |
| SEQ ID NO: 44 | CCTCCTCCGAACTCCG | *PPP2RSC* reverse primer 2 |
| SEQ ID NO: 45 | TTTCGGTATGGGTTTTAGGTC | *PPP2RSC* forward primer 3 |
| SEQ ID NO: 46 | CCTCCTCCGAACTCCG | *PPP2RSC* reverse primer 3 |
| SEQ ID NO: 47 | CGTTGTTTCGTTTCGGAGTTC | *PPP2RSC* forward primer 4 |
| SEQ ID NO: 48 | CAAATCGACGTCCTCCG | *PPP2RSC* reverse primer 4 |
| SEQ ID NO: 49 | TGTTTCGGTTTTCGTTGTTTC | *PPP2RSC* forward primer 5 |
| SEQ ID NO: 50 | CAAATCGACGTCCTCCG | *PPP2RSC* reverse primer 5 |
| SEQ ID NO: 51 | GGAAACGGGAGAGCGC | *PPP2RSC* forward primer 6 |
| SEQ ID NO: 52 | CGCCGATCTAACCTCCTACG | *PPP2RSC* reverse primer 6 |
| SEQ ID NO: 53 | CGGGACGGAAAGGAAAC | *PPP2RSC* forward primer 7 |
| SEQ ID NO: 54 | GCCGATCTAACCTCCTACG | *PPP2RSC* reverse primer 7 |
| SEQ ID NO: 55 | GGTTGGAGGATGAAGTT | *PPP2RSC* forward primer 8 |
| SEQ ID NO: 56 | TCCAACCCTCTACTTAAC | *PPP2RSC* reverse primer 8 |
| SEQ ID NO: 57 | GGTATGGGTTTTAGGT | *PPP2RSC* forward primer 9 |
| SEQ ID NO: 58 | ACTAAAAAATAAAACCCCC | *PPP2RSC* reverse primer 9 |
| SEQ ID NO: 59 | AAGTAGAGGGTTGGAG | *PPP2RSC* forward primer 10 |
| SEQ ID NO: 60 | AAAACCCTTAACTCCC | *PPP2RSC* reverse primer 10 |
| SEQ ID NO: 61 | AGTGTAGAAATTAATAAGTGAGAGGGC | *SDC2* forward primer 1 |
| SEQ ID NO: 62 | TTCCTCCTCCTACGCCTACT | *SDC2* reverse primer 1 |
| SEQ ID NO: 63 | ATAAGTGAGAGGGCGTCGC | *SDC2* forward primer 2 |
| SEQ ID NO: 64 | TTCCTCCTCCTACGCCTACT | *SDC2* reverse primer 2 |
| SEQ ID NO: 65 | CGTTGCGGTATTTTGTTTC | *SDC2* forward primer 3 |
| SEQ ID NO: 66 | AACGAAACCTCCTACCCAA | *SDC2* reverse primer 3 |
| SEQ ID NO: 67 | AAATTAGAAATTGAATTTCGGTACG | *SDC2* forward primer 4 |
| SEQ ID NO: 68 | AACTACTCTCTAAAACTCTTCTTACTCTACT | *SDC2* reverse primer 4 |
| SEQ ID NO: 69 | GCGTTGGGTAGGAGGTTTC | *SDC2* forward primer 5 |
| SEQ ID NO: 70 | ATATTCCCCAAAAACCGACTACT | *SDC2* reverse primer 5 |
| SEQ ID NO: 71 | TCGTTTAGGGTGTTTGAAGTTAC | *SDC2* forward primer 6 |
| SEQ ID NO: 72 | CCAACGAATCTCCGATATATACTT | *SDC2* reverse primer 6 |
| SEQ ID NO: 73 | CGTGGTTTGGGAAGGAC | *SDC2* forward primer 7 |
| SEQ ID NO: 74 | CGAAATAAAACCGTTCCCTC | *SDC2* reverse primer 7 |
| SEQ ID NO: 75 | GGAGTTTGGGTCGGGTTC | *SDC2* forward primer 8 |
| SEQ ID NO: 76 | AAATTTCCAAATCATCCCTAATCTC | *SDC2* reverse primer 8 |
| SEQ ID NO: 77 | GGGAGTGTAGAAATTAATAAGTGAGAGG | *SDC2* forward primer 9 |
| SEQ ID NO: 78 | CTCCTACGCCTACTCCCG | *SDC2* reverse primer 9 |
| SEQ ID NO: 79 | GAGTTCGAGTTTTCGAGTTTGAGT | *SDC2* forward primer 10 |
| SEQ ID NO: 80 | CTACCCAACGCTCGACG | *SDC2* reverse primer 10 |
| SEQ ID NO: 81 | GAGTTCGAGTTTTCGAGTTTGAGT | *SDC2* forward primer 11 |
| SEQ ID NO: 82 | CGAAACCTCCTACCCAACG | *SDC2* reverse primer 11 |
| SEQ ID NO: 83 | GAGTCGGTGAGTGGGTTAGG | *SDC2* forward primer 12 |
| SEQ ID NO: 84 | GCTCCCCTATTCCCTACG | *SDC2* reverse primer 12 |
| SEQ ID NO: 85 | ATATTTTAAACGGCGCGC | *SDC2* forward primer 13 |
| SEQ ID NO: 86 | TAAATCCTACTCACCTTAAACTTAATAACCT | *SDC2* reverse primer 13 |
| SEQ ID NO: 87 | TTGAGGAAGGGAAGAGGG | *SDC2* forward primer 14 |
| SEQ ID NO: 88 | CAATCCCCAAATATACACCG | *SDC2* reverse primer 14 |
| SEQ ID NO: 89 | TTTAGGGATCGGTTGTTTTTAGTC | *SDC2* forward primer 15 |
| SEQ ID NO: 90 | GAATTCGTATACGCGAACTACG | *SDC2* reverse primer 15 |
| SEQ ID NO: 91 | AGTTTAAGGTGAGTAGGATTTACGC | *SDC2* forward primer 16 |
| SEQ ID NO: 92 | TTTTACCCTAATTACAAACAACGAC | *SDC2* reverse primer 16 |
| SEQ ID NO: 93 | GGTTTTTGCGGTTAGATTAAATC | *SDC2* forward primer 17 |
| SEQ ID NO: 94 | AAAAAACGCTACTCGCTAACGT | *SDC2* reverse primer 17 |
| SEQ ID NO: 95 | GTAGGGAATAGGGGAG | *SDC2* forward primer 18 |
| SEQ ID NO: 96 | AAATAACTTCAAAAACCTC | *SDC2* reverse primer 18 |
| SEQ ID NO: 97 | GAAGAAGTTGGGAGTGAT | *SDC2* forward primer 19 |
| SEQ ID NO: 98 | CCTCTTCCCTTCCTCA | *SDC2* reverse primer 19 |
| SEQ ID NO: 99 | CCCAAAACACATTTCTTCCATTC | *GAPDH* probe 1 |
| SEQ ID NO: 100 | AAACGAAGTTTTTTGTTTAGCGTT | *SDC2* probe 1 |
| SEQ ID NO: 101 | GAAAAAACTTCGTTTTACCCTAATTAC | *SDC2* probe 2 |
| SEQ ID NO: 102 | ACACCGAAAATTACGATTTAATCTAAC | *SDC2* probe 3 |
| SEQ ID NO: 103 | ACACCAAAAATTACAATTTAATCTAAC | *SDC2* probe 4 |
| SEQ ID NO: 104 | CTACTCGCTAACGTTACCC | *SDC2* probe 5 |
| SEQ ID NO: 105 | CTACTCACTAACATTACCC | *SDC2* probe 6 |
| SEQ ID NO: 106 | CCTCTTCCCTTCCTCA | *SDC2* probe 7 |
| SEQ ID NO: 107 | TAACGCCAACGAACAACGCTTT | *SDC2* probe 8 |
| SEQ ID NO: 108 | CTAAACCAAACCGCAATTCTCGATA | *SDC2* probe 9 |
| SEQ ID NO: 109 | TAACACCAACAAACAACACTTT | *SDC2* probe 10 |
| SEQ ID NO: 110 | CTAAACCAAACCACAATTCTCAATA | *SDC2* probe 11 |
| SEQ ID NO: 111 | ACCCCGCGATCCGCGAAAATAAAA | *SDC2* probe 12 |
| SEQ ID NO: 112 | CCCGCGATCCGCGAAAATAAAAACACCG | *SDC2* probe 13 |
| SEQ ID NO: 113 | TCACCTATCTAAACCTCAAACCA | *ADHFE1* probe 1 |
| SEQ ID NO: 114 | CCCAACGACGCTCGCAC | *ADHFE1* probe 2 |
| SEQ ID NO: 115 | CAACGACGCTCGCACCAT | *ADHFE1* probe 3 |
| SEQ ID NO: 116 | TAAATAATACGAACGCCGCTAAAAC | *ADHFE1* probe 4 |
| SEQ ID NO: 117 | ATACGAACGCCGCTAAAACAACTAAC | *ADHFE1* probe 5 |
| SEQ ID NO: 118 | CCCAACAACACTCACAC | *ADHFE1* probe 6 |
| SEQ ID NO: 119 | CAACAACACTCACACCAT | *ADHFE1* probe 7 |
| SEQ ID NO: 120 | TAAATAATACAAACACCACTAAAAC | *ADHFE1* probe 8 |
| SEQ ID NO: 121 | ATACAAACACCACTAAAACAACTAAC | *ADHFE1* probe 9 |
| SEQ ID NO: 122 | ACTCAAAACCATTTTCCCACGAAAT | *ADHFE1* probe 10 |
| SEQ ID NO: 123 | AACGAAAAACGCCACTACCCGATACGA | *ADHFE1* probe 11 |
| SEQ ID NO: 124 | AACGCCACTACCCGATACGAACTACACC | *ADHFE1* probe 12 |
| SEQ ID NO: 125 | AAAAACCAAAACGCCAACTACCCCA | *ADHFE1* probe 13 |
| SEQ ID NO: 126 | CCCACGAAATAAAAACCAAAACGCCAAC | *ADHFE1* probe 14 |
| SEQ ID NO: 127 | CAAAACGCCAACTACCCCAACGAC | *ADHFE1* probe 15 |
| SEQ ID NO: 128 | CGAACCGCGCTCTCCC | *PPP2RSC* probe 1 |
| SEQ ID NO: 129 | TTTCCTTTCCGTCCCGTCTCG | *PPP2RSC* probe 2 |
| SEQ ID NO: 130 | AATTAAAACGCGCAACGCAA | *PPP2RSC* probe 3 |
| SEQ ID NO: 131 | CGATTCGCGAACGACC | *PPP2RSC* probe 4 |
| SEQ ID NO: 132 | CTTTACCCCGACCTCCGCTA | *PPP2RSC* probe 5 |
| SEQ ID NO: 133 | CAAACCACACTCTCCC | *PPP2RSC* probe 6 |
| SEQ ID NO: 134 | TTTCCTTTCCATCCCATCTCA | *PPP2RSC* probe 7 |
| SEQ ID NO: 135 | AATTAAAACACACAACACAA | *PPP2RSC* probe 8 |
| SEQ ID NO: 136 | ACACAATTCACAAACAACCA | *PPP2RSC* probe 9 |
| SEQ ID NO: 137 | CTTTACCCCAACCTCCACTA | *PPP2RSC* probe 10 |
| SEQ ID NO: 138 | CCGCCCGCGAACCGCGCTC | *PPP2RSC* probe 11 |
| SEQ ID NO: 139 | TACGCGCTCCAACCCTCTACTTAACCGC | *PPP2RSC* probe 12 |
| SEQ ID NO: 140 | CAACCCTCTACTTAACCGCCCGCGAA | *PPP2RSC* probe 13 |
| SEQ ID NO: 141 | CGTTACGCGCTCCAACCCTCT | *PPP2RSC* probe 14 |
| SEQ ID NO: 142 | TACGTTACGCGCTCCAACCCTCT | *PPP2RSC* probe 15 |

In some embodiments, the reagents for detecting the *ADHFE1* gene include the sequences as set forth in SEQ ID NO: 7, SEQ ID NO: 8, and SEQ ID NO: 127; the reagents for detecting the *PPP2RSC* gene include the sequences as set forth in SEQ ID NO: 53, SEQ ID NO: 54, and SEQ ID NO: 139; the reagents for detecting the *SDC2* gene include the sequences as set forth in SEQ ID NO: 89, SEQ ID NO: 90, and SEQ ID NO: 101; and the composition further includes a reagent for detecting a *GAPDH* gene, the reagent for detecting the *GAPDH* gene including the sequences as set forth in SEQ ID NO: 1, SEQ ID NO: 2, and SEQ ID NO: 99.

The primers or probes mentioned above may be artificially synthesized, for example, using phosphonamidite solid phase support synthesis or other methods commonly used in the related art.

In at least some embodiments, the composition includes at least one selected from PCR buffer, salt ion, dNTP, DNA polymerase, or target nucleic acid. The above-mentioned target nucleic acids can be extracted from the sample by means of some commercially available kits. The target nucleic acid is a nucleic acid sequence containing genes from *ADHFE1, PPP2R5C,* and *SDC2.*

In at least some embodiments, the above-mentioned fluorescent quantitative PCR detection is performed at 95°C for 10 minutes, at 95°C for 15 seconds, at 55°C for 30 seconds, and at 72°C for 30 seconds, for 45 cycles in total.

In at least some embodiments, a mass ratio of the primer set to the probes is 1: 1: 1: 1. Thus, an ideal detection result can be obtained.

In another aspect of the present disclosure, the present disclosure provides a kit for diagnosing colorectal cancer. The kit includes the composition as described above. The kit provided by the present disclosure can specifically detect the methylation modification of *ADHFE1, PPP2R5C,* and *SDC2* genes in a sample, thereby evaluating the risk of colorectal cancer in the subject. In the present disclosure, the combined detection of methylation statuss of three genes, i.e., *ADHFE1, PPP2RSC,* and *SDC2,* can effectively improve the detection performance compared with single gene methylation detection.

In at least some embodiments, the kit further includes at least one of the following reagents: a DNA extraction reagent, a DNA methylation conversion reagent, and a nucleic acid purification reagent. The DNA extraction reagent can be used to obtain DNA in a sample. The DNA methylation conversion reagent can convert cytosine in DNA to uracil. The nucleic acid purification reagent can be used to obtain purified nucleic acids, which can then be used in subsequent reaction processes.

The above-mentioned kit may also be used in the same way as described below, for determining the methylation status of a gene in a sample or assessing the presence of colorectal cancer in a subject. To this end, the present disclosure further provides use of a reagent in the manufacture of a kit for determining methylation status of a gene in a sample. The present disclosure further provides the use of a reagent in the manufacture of a kit for assessing a state of colorectal cancer in a subject.

In yet another aspect, the present disclosure further provides a method for determining methylation status of genes including *ADHFE1* gene, *PPP2R5C* gene, and *SDC2* gene in a sample. The method includes: (1) extracting a genomic DNA from the sample; (2) obtaining a conversion product by converting the genomic DNA with a methylation treatment; and (3) performing, by using a primer set and probes, fluorescent quantitative PCR detection on the conversion product to determine the gene methylation status in the sample.

Generally, the methylation status refers to the presence or absence of 5-methylcytosine in one or more CpG dinucleotides in the DNA sequence. The method for determining the methylation status of genes in the sample, as provided by the present disclosure, can be used as an accurate, simple, and economical means for early screening of colorectal cancer, thereby increasing the detection rate of colorectal cancer, especially early intestinal cancer, in a population with high risk for colorectal cancer and a general physical examination population. In this way, the survival rate of colorectal cancer patients can be increased, and a lot of medical expenses and medical burden can be reduced.

The sample is not particularly limited herein, and the sample may be an *ex vivo* biological sample containing the target nucleic acids, including, but not being limited to, at least one of tissue sample, blood sample, saliva sample, secreta, or excreta. The excreta may be a urine sample, a sweat sample, or a tear sample. In some preferred embodiments, the excreta are a fecal sample. Since the fecal sample contains intestinal exfoliative cells and has non-invasive characteristics for use in the detection, the fecal samples can serve as he preferred test object.

The above-mentioned methylation conversion treatment may be some chemical agents such as sodium bisulfite. As an example, same commercially available kits can be used to perform the methylation conversion treatment, for example, using EZ DNA Methylation-Gold Kit for performing bisulfite conversion on DNA. The obtained product can be subjected to column purification and re-dissolution treatment using a commercial kit to obtain the purified product.

In another aspect of the present disclosure, the present disclosure further provides isolatable nucleic acid sequences including a primer set and probes. The primer set includes: (a) at least one pair of the sequences as set forth in SEQ ID NO: 3 to SEQ ID NO: 40; (b) at least one pair of the sequences as set forth in SEQ ID NO: 41 to SEQ ID NO: 60; and (c) at least one pair of the sequences as set forth in SEQ ID NO: 61 to SEQ ID NO: 98. Probes include: at least one of the sequences as set forth in SEQ ID NO: 100 to SEQ ID NO: 112; at least one of the sequences as set forth in SEQ ID NO: 113 to SEQ ID NO: 127; and at least one of the sequences as set forth in SEQ ID NO: 128 to SEQ ID NO: 142.

The present disclosure further provides a method for assessing a diseased state of colorectal cancer in a subject. The method includes: a) providing a sample of the subject, the sample comprising target nucleic acids of the subject, and the target nucleic acids including nucleic acids from *ADHFE1* gene, *PPP2RSC* gene, and *SDC2* gene; b) assessing a methylation status of the target nucleic acids; and c) assessing the diseased state of colorectal cancer in the subject based on the methylation status of the target nucleic acids. The reagents for assessing the methylation status of the target nucleic acids may be a chemical reagent such as sulfite or hydrosulfite, bisulfite, for example, sodium hydrosulfite. In the target nucleic acids treated with these chemical reagents, the unmethylated cytosine can be converted to uracil, while the methylated cytosine does not undergo such a conversion. Then, the differences of the converted sequences are analyzed through various means, such as sequencing, PCR, high-resolution melting curve analysis, etc., particularly, by means of fluorescent quantitative PCR for quick and easy identification. The reagents for assessing the methylation status of the target nucleic acids may also be some biological reagents, such as polypeptides or enzymes, such as some methylation-sensitive restriction enzymes.

The subject referred to herein is human being, for example be, a patient in need of a diagnosis. The biological samples derived from a subject include, but not limited to, at least one of secreta or excreta such as tissue samples, blood samples, saliva samples, and the like. The excreta may be a urine sample, a sweat sample, or a tear sample. Furthermore, since the fecal sample contains intestinal exfoliative cells and has non-invasive characteristics for use in the detection, the fecal samples can serve as he preferred test object.

In at least some embodiments, the subject is a mammal. In at least some embodiments, the methylation status of the target nucleic acid is assessed in step b) using the above-mentioned primer set and probe.

The method further includes: isolating the target nucleic acids from the sample. The target nucleic acids can be isolated from the sample using commercially available kits known in the related art.

The method further includes: amplifying the target nucleic acids. Common reagents for nucleic acid amplification may be used. Reagents for nucleic acid amplification may include an enzyme, for example, an enzyme that may be used in a polynucleotide amplification reaction such as a fluorescent quantitative polymerase reaction (qPCR).

The method further includes: assessing the methylation status of the target nucleic acids to obtain an indicator of methylation; and comparing the indicator of methylation with a reference value to determine the methylation status of the sample. In some embodiments, the method includes: evaluating the methylation status of the target nucleic acid using a chemical reagent. According to a preferred embodiment of the present disclosure, the chemical reagent includes hydrosulfite or sulfite. In some preferred embodiments, the reference value is derived from an internal reference gene *GAPDH.* The amplified Ct values of the *ADHFE1, PPP2R5C,* and *SDC2* genes are compared with the amplified Ct value of the internal reference *GAPDH* gene to determine the methylation status of the sample gene based on a threshold value. The above-mentioned threshold value may be set based on analysis of a large number of clinical samples of known pathological information. The above-mentioned methylation indicator can also be any other common methylation indicator, such as methylation frequency, methylation haplotype load, etc.

In at least some embodiments, the method further includes: determining whether a DNA content satisfies the detection by using an internal reference gene, and determining the methylation status by using a difference (△Ct) between a Ct value of a target gene and a Ct value of the internal reference gene.

In at least some embodiments, the method further includes: assessing the diseased state of colorectal cancer in the subject by means of a fluorescent quantitative PCR method using *GAPDH* as an internal reference gene; determining, when the Ct value of *GAPDH* is greater than 37, a failure of quality control; determining, when the Ct values of the *ADHFE1, PPP2R5C,* and *SDC2* genes, the diseased state of colorectal cancer in the subject as negative; and calculating, when the Ct values of the *ADHFE1, PPP2RSC,* and *SDC2* genes are ≤ 37, the difference △Ct between the amplified Ct values of *ADHFE1, PPP2RSC,* and *SDC2* genes and the amplified Ct value of internal reference *GAPDH* gene, and evaluating a disease state of colorectal cancer in the subject based on the ROC curve results and AUC results of △Ct of the *ADHFE1, PPP2R5C,* and *SDC2* genes.

The method further includes: determining, when △Ct(*ADHFE1*) ≤ 8, △Ct(*PPP2R5C*) ≤ 5, and △Ct(*SDC2*) ≤ 12, the diseased state of colorectal cancer in the subject as positive.

The method further includes: assessing a likelihood of colorectal cancer based on the methylation status of the subject, thereby assisting the diagnosis and tumor screening to provide the subject with the treatment as early as possible. In at least some embodiments, the treatment includes at least one of chemotherapy, radiation therapy, immunotherapy, cell therapy, and surgery.

The embodiments of the present disclosure will be explained with reference to the following examples. It will be understood by those skilled in the art that the following examples are merely illustrative, and they should not be construed as limitations of the scope of the present disclosure. Specific techniques or conditions that are not specified in the examples shall be those described in the literature in the related art or according to the product description. The used reagents or instruments without indicating the manufacturer are conventional products that can be obtained commercially.

### Example 1

The Applicant identified target nucleic acids for the diagnosis of colorectal cancer according to the following methods and procedures.

Firstly, the Applicant compared the significant differences in methylation levels of different genes in tumor/non-tumor samples in the Cancer Genome Atlas (TCGA) database and screened out the candidate genes for methylation detection, i.e., *CDH4, ZNF132, PPP2R5C, LONRF2, ADHFE1, SDC2, NDRG4,* and *BMP3.*

Secondly, specific primers and probes were designed for these candidate genes. In the process of designing primers and probes, the Applicant found that, due to the methylation of the target nucleic acids, most of cytosines were converted to uracil bases after bisulfite conversion of the genome sequence to be detected, and the genome sequence has low complexity, such that single bases may be continuously present in the design of primers and probes. In addition, annealing temperature was generally low. There were few regions in the primers can be designed.

Thereafter, based on the primers and probes obtained by the above design, the candidate genes were preliminarily screened using a small number of tumor/non-tumor samples. The results of sensitivity/specificity of each gene were as follows: *CDH4* (61.5%/61.5%), *ZNF132* (50%/100%), *PPP2R5C* (78.9%/100%), *LONRF2* (77.8%/72%), *ADHFE1* (100%/91.3%), *SDC2* (88.9%/100%), *NDRG4* (70%/95%), and *BMP3* (65%/90%). Among them, the sensitivity/specificity of *BMP3, NDRG4, ADHFE1, PPP2R5C,* and *SDC2* was better.

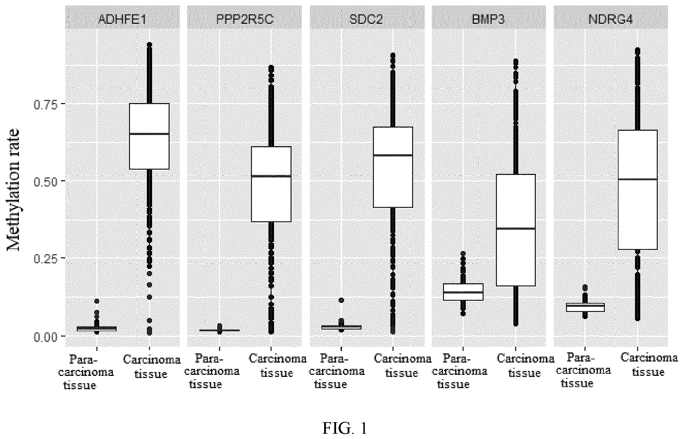

Furthermore, by comparing the methylation data of the above five candidate genes with better sensitivity/specificity in the TCGA database, it was found that, *ADHFE1, PPP2R5C* and *SDC2* genes all exhibited lower background in para-carcinoma tissues than the *BMP3* gene and *NDRG4* gene, and they also exhibited a more significant difference in cancer tissues and para-carcinoma tissues, which are shown in FIG. 1.

Therefore, the Applicant finally determined *ADHFE1, PPP2RSC,* and *SDC2* genes as target nucleic acids for the diagnosis of colorectal cancer, and the Applicant further designed and optimized specific primer and probe sequences for the above target genes, as listed in Table 1.

### Example 2

The Applicant employed the primers and probes for the target nucleic acids (*ADHFE1, PPP2RSC, SDC2* genes) obtained in Example 1 for the diagnosis of colorectal cancer, to amplify the target nucleic acids with the fluorescent quantitative PCR in positive or negative cell lines to verify the effects of primers and probes.

Step 1: sample collection: positive standard DNA (CpGenome Human Methylated DNA Standard Set, Sigma-Aldrich, S8001M); negative standard DNA (CpGenome Human Non-Methylated DNA Standard Set, Sigma-Aldrich, S8001U)

Step 2: DNA modification: 10 ng of each of the above standard DNAs was taken, EZ DNA Methylation-Gold Kit (ZYMO RESEARCH) was used to perform bisulfite conversion on DNA; after column purification, 33 µl of NF water was used to re-dissolved for later use.

### Step 3: PCR detection

For example, by using the primers and probes listed in Table 1, fluorescence quantitative PCR detection was carried out according to the following PCR system and PCR reaction procedure. The primers and probes for *ADHFE1* were selected from SEQ ID NO: 7, SEQ ID NO: 8 and SEQ ID NO: 127. The primers and probes for *PPP2RSC* were selected from SEQ ID NO: 53, SEQ ID NO: 54 and SEQ ID NO: 139. The primers and probes for *SDC2* were selected from SEQ ID NO: 89, SEQ ID NO: 90 and SEQ ID NO: 101. The primers and probes for internal reference *GAPDH* gene were selected from SEQ ID NO: 1, SEQ ID NO: 2, and SEQ ID NO: 99.
5'-end of the probe SEQ ID NO: 127 was FAM, and 3'-end was BHQ1;
5'-end of the probe SEQ ID NO: 139 was CY5, and 3'-end was BHQ3;
5'-end of the probe SEQ ID NO: 101 was VIC, and 3'-end was BHQ1; and
5'-end of the probe SEQ ID NO: 99 was ROX, and 3'-end was MGB.

### (1) TPCR system is shown in Table 2:

**[Table 2] PCR system**

| Component | Final concentration |
|---|---|
| PCR Buffer | 1x |
| Magnesium ion | 2-5 mM |
| dNTP | 0.2∼0.8 mM |
| Primer | 0.2∼0.8 µM |
| Probe | 50∼200 mM |
| Taq Polymerase | 1∼5 U |
| Template | up to 30 ul |

### (2) PCR reaction procedure is shown in Table 3:

**[Table 3] PCR Reaction Procedure**

| Temperature | Time | Number of Cycles |
|---|---|---|
| 95°C | 10min | 1 |
| 95°C | 15s | 45 |
| 55°C | 30s | |
| 72°C | 30s | |

Step 4: result analysis: the difference value (△Ct value) between the amplified Ct values of *ADHFE1, PPP2R5C,* and *SDC2* genes and the amplified Ct value of internal reference *GAPDH* gene was calculated, and it was judged whether the gene methylation degree of the sample is normal based on a judgment threshold, which was set based on the analysis of a large number of clinical samples with known pathological information.

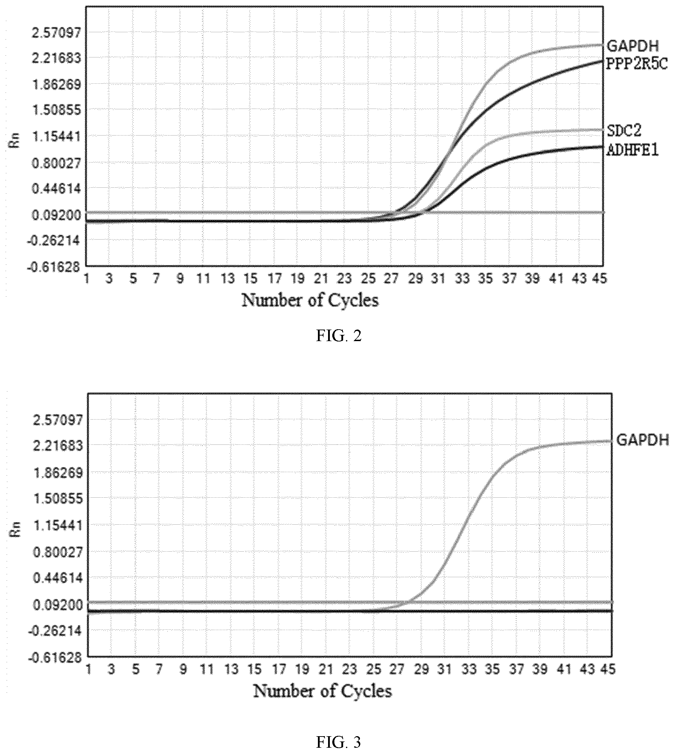

The amplification results of the above primers and probes are shown in FIG. 2 and FIG. 3.

### Example 3

The present Applicant have designed and developed a kit for diagnosing colorectal cancer. The kit includes specific primers and probes for specifically detecting methylation of the *ADHFE1* gene, *PPP2R5C* gene, and *SDC2* gene. The specific primers contained in different kits are selected from the primers listed in Table 1, and the probes are selected from the probes listed in Table 1. Each kit includes at least one pair of forward and reverse primers for specifically detecting the *ADHFE1* gene, at least one pair of forward and reverse primers for specifically detecting the *PPP2R5C* gene, and at least one pair of forward and reverse primers for specifically detecting the *SDC2* gene. In addition, the kit may further include at least one probe for detecting the *ADHFE1* gene, at least one probe for detecting the *PPP2RSC* gene and at least one probe for detecting the *SDC2* gene, as required.

The developed kit may further include forward and reverse primers and probes for detecting an internal reference gene, such as *GAPDH,* as required.

5'-end of the probe may be attached to a fluorophore such as FAM, VIC, ROX, or CY5, and 3'-end thereof may be attached to a quencher such as MGB.

The primer and probe sequences in the kit may be provided in powder form.

In addition, the kit may further contain PCR reaction buffer, polymerase, DNA methylation conversion reagent, etc. as required, thereby facilitating the diagnosis of colorectal cancer by those skilled in the art with the method according to the present disclosure.

### Example 4

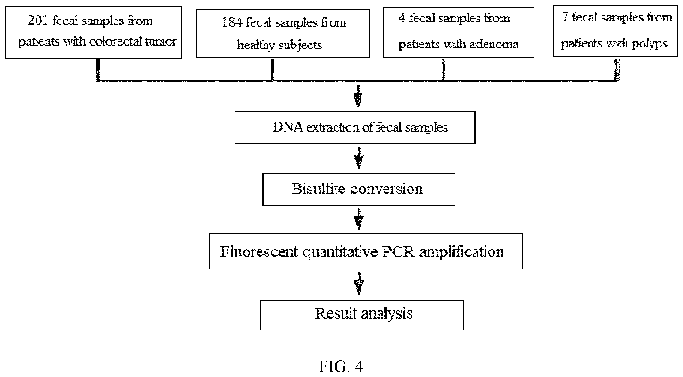

The Applicant employed the kit provided in Example 3 for diagnosing colorectal cancer, in which the methylation of the sample was detected with the following method including steps illustrated in FIG. 4.
1. Sample collection

A total of 201 colorectal cancer samples, 184 healthy controls, 4 adenoma samples, and 7 polyp samples were collected.

### 2. Sample DNA extraction

The collected samples were subjected to DNA extraction using a commercial fecal sample DNA extraction kit.

### 3. DNA modification

The DNA was subjected to bisulfite conversion by using EZ DNA Methylation-Gold Kit (ZYMO RESEARCH), column purification, and re-dissolution for later use.

### 4. PCR detection

In this example, the primers and probes for *ADHFE1* were selected from SEQ ID NO: 7, SEQ ID NO: 8, and SEQ ID NO: 127; the primers and probes for *PPP2R5C* were selected from SEQ ID NO: 53, SEQ ID NO: 54, and SEQ ID NO: 139; the primers and probes for *SDC2* were selected from SEQ ID NO: 89, SEQ ID NO: 90, and SEQ ID NO: 101; the primers and probes for internal reference *GAPDH* gene were selected from SEQ ID NO: 1, SEQ ID NO: 2, and SEQ ID NO: 99. The collected samples were subjected to fluorescent quantitative PCR detection according to the PCR system and reaction conditions as described in Example 2.

### 5. Results analysis

The detection was performed by using SLAN 96S qPCR instrument. The baseline and threshold values were set as default. The threshold value of the Ct value of each gene was 37.

When the Ct value of *GAPDH* was greater than 37, it was judged as a failure of quality control.

When the *ADHFE1, PPP2R5C,* and *SDC2* genes were greater than 37, it was judged as negative.

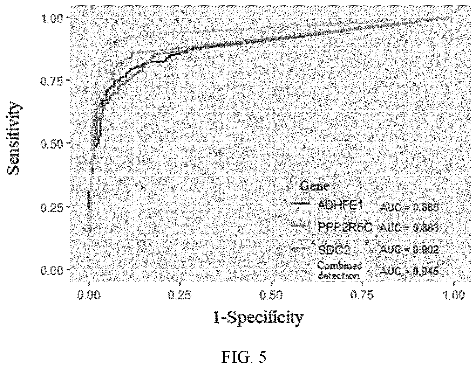

When the Ct values of *ADHFE1, PPP2R5C,* and *SDC2* genes were ≤ 37, the difference (△Ct value) between the amplified Ct values of *ADHFE1, PPP2R5C* and *SDC2* genes and the amplified Ct value of internal reference *GAPDH* gene was calculated. SPSS software was used to plot ROC curves of △Ct values of the three genes to be detected. The ROC curves are shown in FIG. 5, and the performances of the respective genes and the area under the curve (AUC) are shown in Table 4.

**[Table 4] Performance of each gene and area under the curve (AUC)**

| Gene | AUC | Threshold | SEN | SPE |
|---|---|---|---|---|
| ΔCt_*ADHFE1* | 0.886 | 11.04 | 79.10% | 88.60% |
| ΔCt_*PPP2R5C* | 0.883 | 12.66 | 83.60% | 83.20% |
| ΔCt_*SDC2* | 0.902 | 11.15 | 81.10% | 92.40% |

In order to further improve the performance of the kit, △Ct values of the three genes were integrated for analysis, and the AUC could reach 0.945. Finally, △Ct(*ADHFE1*) ≤ *8,* △Ct(*PPP2R5C*) ≤ 5, and △Ct(*SDC2*) ≤ 12 were determined to be positive for the genes. When any gene was positive, the sample was determined to be positive. Under this condition, when the specificity was 94.02%, the sensitivity could reach 87.56%.

Among them, the sensitivity is used to indicate a percentage of positive samples diagnosed as colorectal cancer in clinicopathological diagnosis or patients diagnosed as colorectal cancer through detection.

The specificity refers to a rate of a sample diagnosed as normal in a clinicopathological diagnosis or a patient finally diagnosed as normal.

The above results indicate that, through the combined detection of multiple genes, the present disclosure can obtain better detection performance than single gene detection.

In the specification, references to descriptions of the terms "one embodiment", "some embodiments", "examples", "specific examples", or "some examples", etc. mean that a particular feature, structure, material, or characteristic described in connection with the embodiment or example is included in at least one embodiment or example of the present disclosure. In this specification, schematic representations of the above terms are not necessarily directed to the same embodiment or example. Furthermore, the particular features, structures, materials, or characteristics described may be combined in any suitable manner in any one or more embodiments or examples. Furthermore, combinations and combinations of the various embodiments or examples and features of the various embodiments or examples described in this specification can be made by those skilled in the art without departing from the scope of the present disclosure.

The embodiments of the present disclosure are illustrated and described above. It can be understood that the above-described embodiments are illustrative and not restrictive, and that those skilled in the art can make changes, modifications, substitutions, and alterations thereto without departing from the scope of the present disclosure.

## Claims

1. A composition for detecting colorectal cancer, comprising reagents for detecting methylation status of *ADHFE1* gene, *PPP2R5C* gene, and *SDC2* gene.

2. The composition according to claim 1, wherein the reagents comprise a primer set and probes,
wherein the primer set comprises:
(a-1) at least one of sequences as set forth in SEQ ID NO: 3 to SEQ ID NO: 40;
(b-1) at least one of sequences as set forth in SEQ ID NO: 41 to SEQ ID NO: 60; and
(c-1) at least one of sequences as set forth in SEQ ID NO: 61 to SEQ ID NO: 98,
wherein the probes comprise:
(a-2) at least one of sequences as set forth in SEQ ID NO: 100 to SEQ ID NO: 112;
(b-2) at least one of sequences as set forth in SEQ ID NO: 113 to SEQ ID NO: 127; and
(c-2) at least one of sequences as set forth in SEQ ID NO: 128 to SEQ ID NO: 142.

3. The composition according to claim 2, wherein:
the primer set further comprises sequences as set forth in SEQ ID NO: 1 and SEQ ID NO: 2; and
the probes further comprise a sequence as set forth in SEQ ID NO: 99.

4. The composition according to claim 1, wherein:
the reagent for detecting the *ADHFE1* gene comprises sequences as set forth in SEQ ID NO: 7, SEQ ID NO: 8, and SEQ ID NO: 127;
the reagent for detecting the *PPP2R5C* gene comprises sequences as set forth in SEQ ID NO: 53, SEQ ID NO: 54, and SEQ ID NO: 139; and
the reagent for detecting the *SDC2* gene comprises sequences as set forth in SEQ ID NO: 89, SEQ ID NO: 90, and SEQ ID NO: 101,
optionally, the composition further comprises a reagent for detecting a *GAPDH* gene, the reagent for detecting the *GAPDH* gene comprising sequences as set forth in SEQ ID NO: 1, SEQ ID NO: 2, and SEQ ID NO: 99.

5. The composition according to claim 1, further comprising at least one selected from PCR buffer, salt ion, dNTP, DNA polymerase, or target nucleic acid.

6. A kit for diagnosing colorectal cancer, the kit comprising the composition according to any one of claims 1 to 5.

7. The kit according to claim 6, further comprising at least one of the following reagents:
a DNA extraction reagent, a DNA methylation conversion reagent, and a nucleic acid purification reagent.

8. A method for determining methylation status of genes in a sample, wherein the genes comprise an *ADHFE1* gene, a *PPP2R5C* gene, and an *SDC2* gene,
the method comprising:
(1) extracting genomic DNA from a biological sample;
(2) obtaining the conversion product by treating the genomic DNA wherein a methylated site exhibits a difference from non-methylated site through the methylation treatment of the genomic DNA; and
(3) performing, by using a primer set and probes, fluorescent quantitative PCR detection on the conversion product to determine the methylation status the genes in the sample.

9. The method according to claim 8, wherein the sample comprises at least one selected from a tissue sample, a blood sample, secreta, or excreta.

10. The method according to claim 9, wherein the excreta comprises fecal sample.

11. The method according to claim 8, wherein the primer set comprises:
(a) at least one of sequences as set forth in SEQ ID NO: 3 to SEQ ID NO: 40;
(b) at least one of sequences as set forth in SEQ ID NO: 41 to SEQ ID NO: 60; and
(c) at least one of sequences as set forth in SEQ ID NO: 61 to SEQ ID NO: 98;
wherein probes comprise:
at least one of sequences as set forth in SEQ ID NO: 100 to SEQ ID NO: 112;
at least one of sequences as set forth in SEQ ID NO: 113 to SEQ ID NO: 127; and
at least one of sequences as set forth in SEQ ID NO: 128 to SEQ ID NO: 142.

12. The method according to claim 8, wherein the fluorescent quantitative PCR detection is performed at 95°C for 10 minutes, at 95°C for 15 seconds, at 55°C for 30 seconds, and at 72°C for 30 seconds, for 45 cycles in total.

13. Isolated nucleic acid sequences, comprising a primer set and probes,
wherein the primer set comprises:
(a) at least one of sequences as set forth in SEQ ID NO: 3 to SEQ ID NO: 40;
(b) at least one of sequences as set forth in SEQ ID NO: 41 to SEQ ID NO: 60; and
(c) at least one of sequences as set forth in SEQ ID NO: 61 to SEQ ID NO: 98;
wherein probes comprise:
at least one of sequences as set forth in SEQ ID NO: 100 to SEQ ID NO: 112;
at least one of sequences as set forth in SEQ ID NO: 113 to SEQ ID NO: 127; and
at least one of sequences as set forth in SEQ ID NO: 128 to SEQ ID NO: 142.

14. A method for assessing a diseased state of colorectal cancer in a subject, the method comprising:
a) providing a sample of the subject, the sample comprising target nucleic acids of the subject, and the target nucleic acids comprising nucleic acids from *ADHFE1* gene, *PPP2R5C* gene, and *SDC2* gene;
b) assessing a methylation status of the target nucleic acids; and
c) assessing the diseased state of colorectal cancer in the subject based on the methylation status of the target nucleic acids.

15. The method according to claim 14, wherein the subject is a mammal.

16. The method according to claim 14, wherein the methylation status of the target nucleic acids is assessed in step b) by using a primer set and probes,
wherein the primer set comprises:
(a) at least one of sequences as set forth in SEQ ID NO: 3 to SEQ ID NO: 40;
(b) at least one of sequences as set forth in SEQ ID NO: 41 to SEQ ID NO: 60; and
(c) at least one of sequences as set forth in SEQ ID NO: 61 to SEQ ID NO: 98,
wherein probes comprise:
at least one of sequences as set forth in SEQ ID NO: 100 to SEQ ID NO: 112;
at least one of sequences as set forth in SEQ ID NO: 113 to SEQ ID NO: 127; and
at least one of sequences as set forth in SEQ ID NO: 128 to SEQ ID NO: 142.

17. The method according to claim 14, further comprising:
isolating the target nucleic acids from the sample.

18. The method according to claim 14, further comprising:
amplifying the target nucleic acids.

19. The method according to claim 14, further comprising:
determining whether a DNA content satisfies the detection by using an internal reference gene, and determining the methylation status by using a difference (△Ct) between a Ct value of a target gene and a Ct value of the internal reference gene.
